# EUROPEAN PATENT APPLICATION

(11) **EP 2 092 884 A1**
(43) Date of publication of application: **26.08.2009**
(21) Application number: 06834625.3
(22) Date of filing: 13.12.2006
(51) Int. Cl.: A61B 5/055, A61B 6/00, A61B 6/03, A61B 19/00

(54) **MEDICAL IMAGING MARKER**

(71) Applicant: Imagnosis Inc., Kobe-shi Hyogo 658-0032 (JP)
(72) Inventor: KIM, Han-Joon, Kobe-shi Hyogo 658-0032 (JP)
(74) Representative: Steil, Christian
(86) International application number: PCT/JP2006/324870
(87) International publication number: WO 2008/072323

(57) **Abstract**

An inventive medical imaging marker includes two plate members (2a, 2b) of an imageable material, which respectively includes: two flat major surfaces (4a, 4b) located symmetrically about an intersection (7) of two orthogonal straight lines (5, 6); and two pairs of side surfaces (8a, 9a; 8b, 9b) disposed perpendicularly to the respective major surfaces (4a, 4b) with their boundary edges (5a, 6a; 5b, 6b) defined by at least parts of the straight lines (5, 6). With the use of the marker, an image can be easily and accurately correlated with an actual entity.

## Description

### TECHNICAL FIELD

The present invention relates to a medical imaging marker to be used in imaging a patient by a medical imaging apparatus such as CT or MRI.

### BACKGROUND ART

In the medical field, when a medical treatment is carried out based on the results of image diagnosis or simulation performed with the use of medical three-dimensional image information obtained through imaging by means of an imaging apparatus such as CT or MRI, a treatment position and a treatment direction specified on an image should be mapped onto an actual entity.

A dental implant (artificial tooth root) implantation process will be described by way of example.

Instead of an X-ray photograph (conventional two-dimensional image), 3D-CT data (CT imaging data) including accurate three-dimensional positional information is utilized in image diagnosis for determining an implantation position and an implantation direction. For example, a resin base (generally referred to as "diagnostic stent") is produced by preparing a dummy tooth to be provided at a tooth deficient site, drilling the dummy tooth in an assumptive implant implantation direction, and injecting an imageable material into the resulting hole. With the diagnostic stent being fitted in a patient's oral cavity, the CT imaging is performed. Thus, a guide channel (a filler in the hole) is displayed on the resulting three-dimensional image to indicate the assumptive implantation position and direction.

With reference to the guide channel, tomographic images of a jaw bone are formed, and the implantation position and a portion to be treated are confirmed and diagnosed based on the tomographic images. If there is no problem with the assumptive position and direction as the result of the diagnosis, the diagnostic stent is used as it is as a guide for the treatment. In the treatment, the jaw bone is drilled along the guide channel, and an implant is implanted into the jaw bone.

In this manner, the results of the image diagnosis are mapped onto the actual entity to define the treatment position and direction (see Patent Documents 1 and 2).
Patent Document 1: JP-A-2003-245289
Patent Document 2: JP-A-2001-212158

### DISCLOSURE OF THE INVENTION

### PROBLEMS TO BE SOLVED BY THE INVENTION

In practice, however, the implantation position and direction often require alteration from the assumptive implantation position and direction defined by the guide channel. In this case, it is difficult to accurately map the altered position and direction onto the diagnostic stent with reference to the guide channel alone.

To cope with this, an attempt is made to determine the three-dimensional positional coordinates of the implantation position and direction determined in the image diagnosis and to prepare a guide through CAD based on the positional coordinates. However, the CT imaging is typically capable of providing data of images captured at an interval of 0.3 to 1 mm because of a problem associated with an exposure dose. In the dental field, it is difficult to produce a correct guide directly from the CT data through the CAD because of an artifact (image noise) caused by a metal prosthesis.

Therefore, it is necessary to utilize a dental arch model obtained in an oral cavity. An exemplary method of utilizing the dental arch model is to obtain three-dimensional geometrical information from the dental arch model by a laser scanner or the like, and then produce a guide through the CAD based on positional coordinates obtained as a result of the image diagnosis on an image obtained by replacing the corresponding dental arch region of the 3D-CT image with the dental arch model geometrical information or on the dental arch model geometrical information.
Alternatively, a method of producing the guide directly on the dental arch model or modifying the diagnostic stent based on positional coordinate information obtained as a result of the image diagnosis with the use of a three-dimensional measurement apparatus or the like capable of measuring and displaying three-dimensional positional coordinates.

In any of the aforementioned methods, the accuracy of a marker (a mark defining a position) on the stent for correlating the actual entity with the image is important in order to accurately map the position and the direction specified on the image onto the actual entity.

Conventionally, spherical markers are employed in most cases. However, such a spherical marker defines a single specific point by its center. Therefore, at least three spherical markers are required for three-dimensionally correlating the positional information. Since the specific point is present inside the marker, it is difficult to define the specific point from the surface by the three-dimensional measurement apparatus or the like.

In the medical field, when the medical treatment is carried out based on the results of the image diagnosis or the simulation performed with the use of the medical three-dimensional image information obtained through imaging by means of the imaging apparatus such as CT or MRI , the treatment position and direction specified on the image should be mapped onto the actual entity. Therefore, a medical imaging marker is demanded, which is capable of easily and accurately determining a three-dimensional positional relationship between the three-dimensional image and the actual entity.

In view of the foregoing, it is an object of the present invention to provide a medical imaging marker which is capable of easily and accurately correlating an image with an actual entity.

### MEANS FOR SOLVING THE PROBLEMS

According to an inventive aspect as set forth in claim 1, there is provided a medical imaging marker including a plate member of an imageable material which includes: two flat major surfaces located symmetrically about an intersection of two orthogonal straight lines and each having edges defined by the straight lines; and two pairs of side surfaces disposed perpendicularly to the respective major surfaces with their boundary edges defined by at least parts of the straight lines.

According to an inventive aspect as set forth in claim 2, the medical imaging marker of claim 1 further includes a complementary member of a non-imageable material which fills a region in which the two major surfaces are absent and has a flat surface continuous to the two major surfaces.

According to an inventive aspect as set forth in claim 3, the complementary member entirely covers a back surface of the plate member of the imageable material opposite from the two major surfaces in the medical imaging marker of claim 2.

According to an inventive aspect as set forth in claim 4, the medical imaging marker of claim 1 or 2 has a rectangular contour as a whole as seen from above the major surfaces perpendicularly to the major surfaces.

According to an inventive aspect as set forth in claim 5, the medical imaging marker of claim 2 or 3 has a round contour as a whole as seen from above the major surfaces perpendicularly to the major surfaces.

According to an inventive aspect as set forth in claim 6, the plate member includes a connection portion which connects the two major surfaces in a region including the intersection of the two straight lines in the medical imaging marker of any of claims 1 to 5.

According to an inventive aspect as set forth in claim 7, the back surface opposite from the major surfaces has a given non-planar geometry in the medical imaging marker of any of claims 1 to 6.

According to an inventive aspect as set forth in claim 8, there is provided a planar medical imaging marker entirely composed of an imageable material and including a flat major surface having at least two orthogonal edges, and a pair of side surfaces extending perpendicularly from the two orthogonal edges of the major surface with their boundary edges defined by the two orthogonal edges.

### EFFECTS OF THE INVENTION

According to the present invention, the position and the orientation of a desired part can be accurately determined on a medical three-dimensional image based on an image of the plate member of the imageable material. Further, the position and the orientation determined on the image can be accurately mapped onto an actual entity with reference to the inventive marker.

According to the present invention, more specifically, where the side surfaces are viewed from above the major surfaces or in a viewing direction perpendicular to the major surfaces, for example, edges of the side surfaces adjacent to the major surfaces are seen as respectively aligned with edges of the side surfaces opposite from the major surfaces. Therefore, continuous boundary surfaces defined in association with the two major surfaces are seen as continuous straight lines, making it easy to define coordinate axes including an X-axis and a Y-axis. Where the viewing direction is not perpendicular to the major surfaces, on the other hand, the side surfaces are each displayed as a plane. Therefore, boundary surfaces defined in association with one of the major surfaces are displayed as each having a greater area (the edges of the side surfaces opposite from the one major surface are displayed as boundary lines of a contrast image), and only the edges of the side surfaces adjacent to the other major surface are displayed (the edges opposite from the other major surface are located behind with respect to the viewing direction and hence hidden). Accordingly, the continuous boundary surfaces defined in association with the two major surfaces are not displayed as straight lines. Thus, errors occurring in setting the viewing direction and the coordinate axes (or coordinate planes) which are important for defining the coordinate system based on the marker can be easily detected based on whether or not the continuous boundary surfaces defined between the imageable structural portion and the non-imageable structural portion are displayed as the straight lines.

With the inventive marker, which includes the three-dimensionally continuous boundary surfaces defined by the geometry and the arrangement of orthogonal planar surface portions including the major surfaces and the side surfaces, a marker image can be accurately and easily correlated with the actual entity of the marker.

This makes it possible to more accurately and easily correlate two or more medical three-dimensional images captured at different times by means of different imaging apparatuses with each other or to correlate the position and the orientation of a part specified on the medical three-dimensional image with the position and the orientation of a part specified on the actual entity. Further, it is possible to accurately map the position and the orientation specified on the image onto the actual entity, and vice versa.

### BRIEF DESCRIPTION OF THE DRAWINGS

Figs. 1(a) to 1(d) are diagrams for explaining a medical imaging marker 1 according to one embodiment of the present invention.
Figs. 2(a) to 2(d) are diagrams for explaining a medical imaging marker 11 according to another embodiment of the present invention.
Figs. 3(a) to 3(d) are diagrams for explaining a medical imaging marker 21 according to further another embodiment of the present invention.
Figs. 4(a) to 4(d) are diagrams for explaining a medical imaging marker 31 according to still another embodiment of the present invention.
Figs. 5(a) to 5(d) are diagrams for explaining a medical imaging marker 41 according to further another embodiment of the present invention.
Figs. 6(a) to 6(d) are diagrams for explaining a medical imaging marker 51 according to still another embodiment of the present invention.
Figs. 7(a) to 7(d) are diagrams for explaining a medical imaging marker 61 according to further another embodiment of the present invention.
Figs. 8(a) to 8(c) are diagrams for explaining the fact that back surfaces of the medical imaging markers 1, 31, 41 can each have a given non-planar geometry.
Figs. 9(a) to 9(d) are diagrams for explaining a medical imaging marker 71 according to still another embodiment of the present invention.
Figs. 10(a) to 10(d) are diagrams for explaining a medical imaging marker 81 according to further another embodiment of the present invention.
Figs. 11(a) to 11(d) are diagrams for explaining a medical imaging marker 91 according to still another embodiment of the present invention.
Figs. 12(a) and 12(b) are diagrams for explaining how to achieve image positioning based on a marker image.
Figs. 13(a) and 13(b) are diagrams for explaining how to achieve the image positioning based on the marker image.
Figs. 14(a) to 14(c) are diagrams for explaining another method of defining a coordinate system based on the marker image.
Fig. 15 is a perspective view illustrating a stent 12 fitted on an actual entity (dental arch model) and marked with the medical imaging marker 31 according to the embodiment of the present invention.
Fig. 16 is a diagram of a three-dimensional image based on CT imaging information of Fig. 15.
Fig. 17 is a diagram for explaining how to map an implant implantation position and direction onto the stent 12.
Fig. 18 is a diagram illustrating one example of a produced guide.

### BRIEF DESCRIPTION OF REFERENCE CHARACTERS

- 1,11,21,31,41,51,61,71,81,91:: Medical imaging markers
- 2a,2b:: Plate members
- 3a,3b:: Complementary members
- 4a,4b:: Major surfaces
- 5a,5b:: Edges (straight line)
- 6a,6b:: Edges (straight line)
- 7:: Corner
- 8a,8b,9a,9b:: Side surfaces
- 42:: Connection portions

### EMBODIMENTS OF THE INVENTION

Embodiments of the present invention will hereinafter be described more specifically with reference to the drawings.

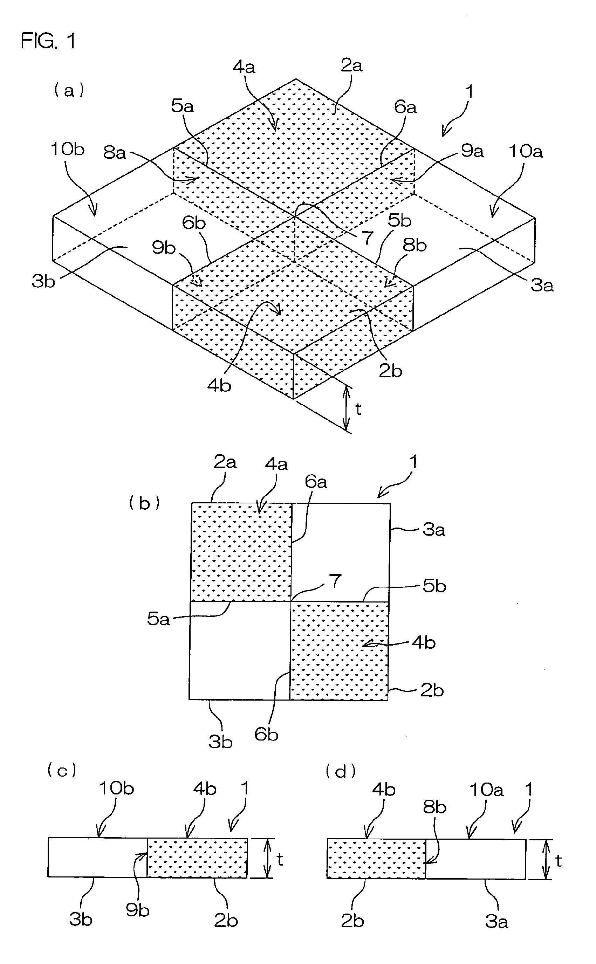

Figs. 1(a), 1(b), 1(c) and 1(d) are a perspective view, a plan view, a front view and a right side view, respectively, of a medical imaging marker (hereinafter referred to simply as "marker") 1 according to one embodiment of the present invention.

Referring to Figs. 1(a) to 1(d), the marker 1 is such that two thin planar rectangular-parallelepiped plate members 2a, 2b each having a square plan shape and a thickness t and two thin planar rectangular-parallelepiped complementary members 3a, 3b each having a thickness t are combined with one another in a checker pattern and, therefore, has a rectangular-parallelepiped form having a square plan shape as a whole.

The plate members 2a, 2b, which are shaded, are composed of an imageable material (e.g., aluminum, apatite or the like) so as to provide an opaque image when being imaged by means of a medical imaging apparatus such as CT or MRI.

On the other hand, the complementary members 3a, 3b are composed of a non-imageable material (e.g., an acryl resin or the like) which is transmissive and provides no image when being imaged by means of the medical imaging apparatus.

The plate members 2a, 2b are disposed with their corners 7 opposed to each other, so that an edge 5a of the contour of an upper surface (major surface) 4a of the plate member 2a and an edge 5b of the contour of an upper surface (major surface) 4b of the plate member 2b are aligned with each other to define a single straight line as seen in plan. Further, another edge 6a of the contour of the major surface 4a and another edge 6b of the contour of the major surface 4b are also aligned with each other to define a single straight line as seen in plan. The plate members 2a, 2b each have a rectangular-parallelepiped form, so that the major surfaces 4a, 4b thereof are naturally planar. Therefore, the two major surfaces 4a, 4b are horizontally arranged to be flush with each other.

In other words, the marker 1 having the aforementioned construction is as follows. The marker 1 has two flat major surfaces 4a, 4b located symmetrically about an intersection 7 of two orthogonal straight lines 5 (5a+5b) and 6 (6a+6b), and two pairs of side surfaces 8a, 9a and 8b, 9b disposed perpendicularly to the respective major surfaces 4a, 4b with their boundary edges 5a, 6a and 5b, 6b defined by at least parts of the straight lines 5, 6, and includes a first plate member 2a including the major surface 4a and the side surfaces 8a, 9a and composed of an imageable material, and a second plate member 2b including the major surface 4b and the side surfaces 8b, 9b and composed of the imageable material.

The complementary members 3a, 3b fill regions in which the two major surfaces 4a, 4b are absent, and respectively have flat surfaces 10a, 10b horizontally continuous to the two major surfaces 4a, 4b.

The side surfaces 8a, 9a are perpendicular to the major surface 4a, and the side surfaces 8b, 9b are perpendicular to the major surface 4b, because the plate members 2a, 2b each have a rectangular-parallelepiped form.

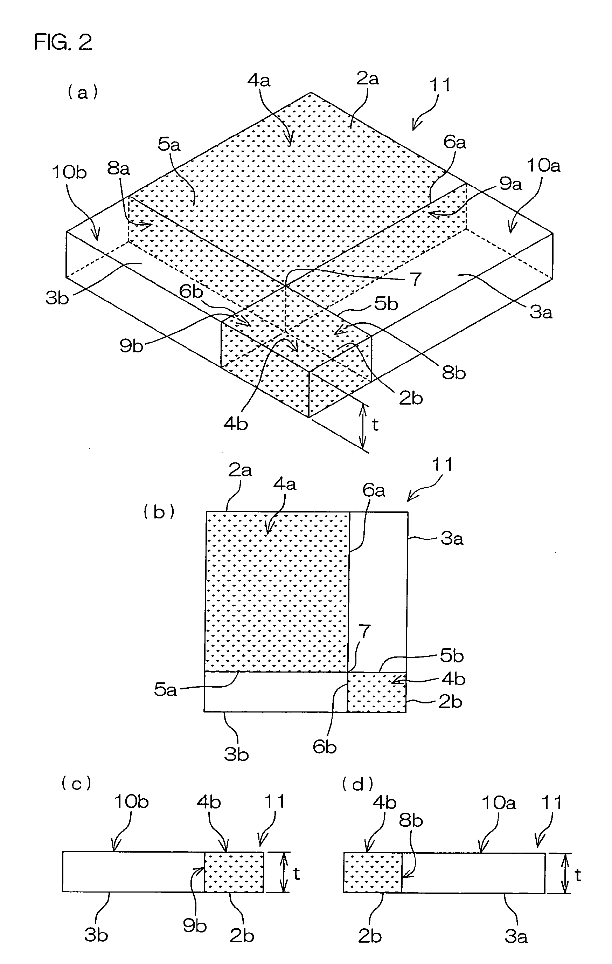

Figs. 2(a), 2(b), 2(c) and 2(d) are a perspective view, a plan view, a front view and a right side view, respectively, of a marker 11 according to another embodiment of the present invention. The marker 11 shown in Figs. 2(a) to 2(d) are different from the marker 1 shown in Figs. 1(a) to 1(d) in that the plate members 2a, 2b are different in size as seen in plan, through the marker 11 has a square plan shape as a whole. That is, the plate member 2a has a rectangular-parallelepiped form having a relatively great major surface 4a, and the plate member 2b has a rectangular-parallelepiped form having a relatively small major surface 4b. Further, the complementary members 3a, 3b fill regions in which the two major surfaces 4a, 4b are absent, so that the marker 11 has a square plan shape as a whole.

The other construction of the marker 11 is the same as that of the marker 1 shown in Figs. 1(a) to 1(d). Therefore, like components are denoted by like reference characters, and duplicate description is omitted.

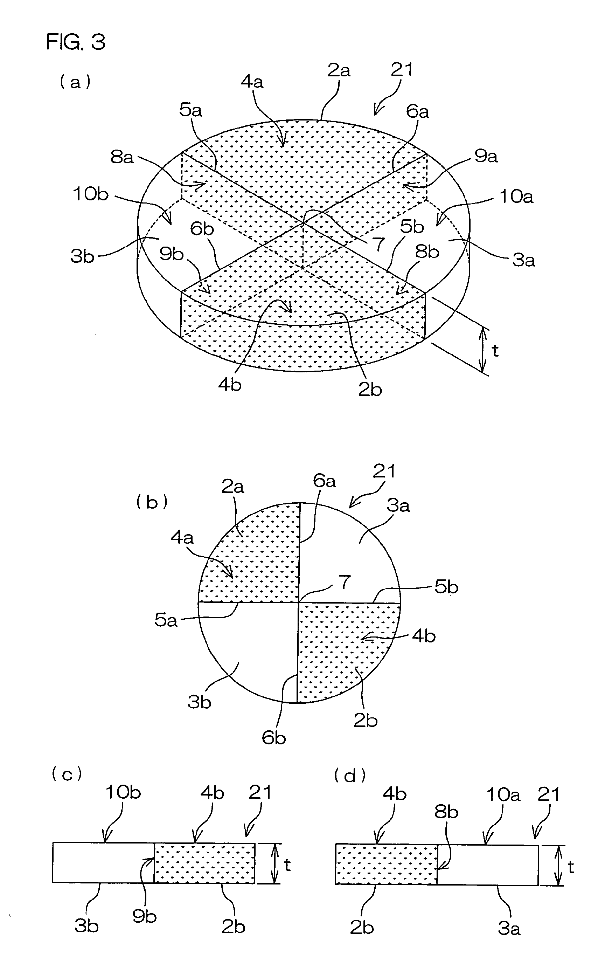

Figs. 3(a), 3(b), 3(c) and 3(d) are a perspective view, a plan view, a front view and a right side view, respectively, of a marker 21 according to further another embodiment of the present invention. The marker 21 shown in Figs. 3(a) to 3(d) are different from the marker 1 shown in Figs. 1(a) to 1(d) in that the marker 21 has a round contour (outer shape) as seen in plan and, therefore, the plate members 2a, 2b and the complementary members 3a, 3b each have a fan plan shape and a thickness t.

In the marker 21 shown in Figs. 3(a) to 3(d), the major surfaces 4a, 4b are horizontally flush with each other. The edges 5a, 5b are aligned on a straight line, and the edges 6a, 6b are aligned on another straight line. These lines 5 (5a+5b) and 6 (6a+6b) orthogonally intersect each other, and the two major surfaces (planar surfaces) 4a, 4b are disposed on opposite sides of the intersection 7.

The side surfaces 8a, 9a are disposed perpendicularly to the major surface 4a with their boundary edges defined by the edges 5a, 6a. The side surfaces 8b, 9b are disposed perpendicularly to the major surface 4b with their boundary edges defined by the edges 5b, 6b.

The plate member 2a, which has the major surface 4a and the two side surfaces 8a, 9a, is composed of an imageable material. Similarly, the plate member 2b, which has the major surface 4b and the two side surfaces 8b, 9b, is composed of an imageable material.

The complementary members 3a, 3b, which fill the regions in which the major surfaces 4a, 4b are absent, are composed of a non-imageable material.

The contour (outer shape) of the marker 21 of Figs. 3(a) to 3(d) as seen in plan is not limited to the round shape, but may be of a rhombic shape, an elliptical shape or any other shape.

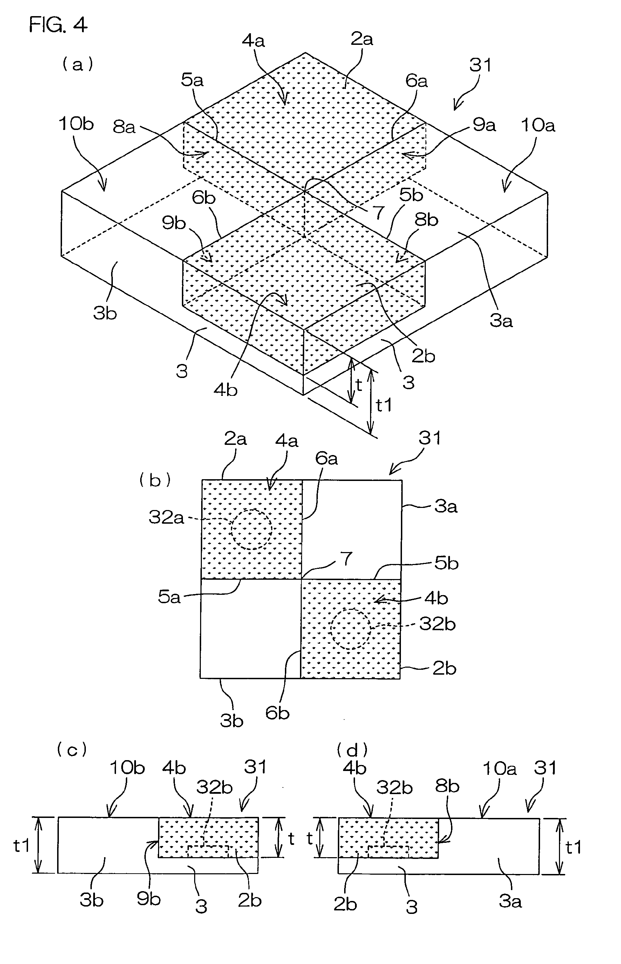

Figs. 4(a), 4(b), 4(c) and 4(d) are a perspective view, a plan view, a front view and a right side view, respectively, of a marker 31 according to still another embodiment of the present invention.

The marker 31 shown in Figs. 4(a) to 4(d) are different from the marker 1 shown in Figs. 1(a) to 1(d)
in that a complementary member 3 covers rear surfaces of the plate members 2a, 2b of the imageable material (opposite from the major surfaces 4a, 4b). In other words, the marker 31 is configured such that a planar complementary member 3 having a square plan shape and composed of a non-imageable material is bonded to the entire bottom of the marker 1 shown in Figs. 1(a) to 1(d), and unified with the complementary members 3a, 3b. The plate members 2a, 2b each have a thickness t, but the marker 31 has an overall thickness t1 (t1>t).

The marker 1 shown in Figs. 1(a) to 1(d) are produced by bonding the side surfaces of the plate members 2a, 2b to the side surfaces of the complementary members 3a, 3b with a non-imageable adhesive agent.
However, this operation is troublesome.

The marker 31 shown in Figs. 4(a) to 4(d) can be produced, for example, by placing the two plate members 2a, 2b at predetermined positions in a mold and then filling the non-imageable material in portions of the mold below the two plate members and portions of the mold in which the two major surfaces 4a, 4b are absent. This facilitates the production operation.

The marker 31 may have fixing recesses 32a, 32b provided in rear surfaces of the plate members 2a, 2b (opposite from the major surfaces 4a, 4b) for receiving the complementary member 3 for proper bonding between the plate members 2a, 2b and the complementary member 3.

This is because only the major surfaces 4a, 4b, the side surfaces 8a, 8b, 9a, 9b and the edges 5a, 6a, 5b, 6b defining the straight lines are essentially utilized in the captured image of the plate members 2a, 2b as will be described below and, therefore, the other portion of the marker may have a desired shape.

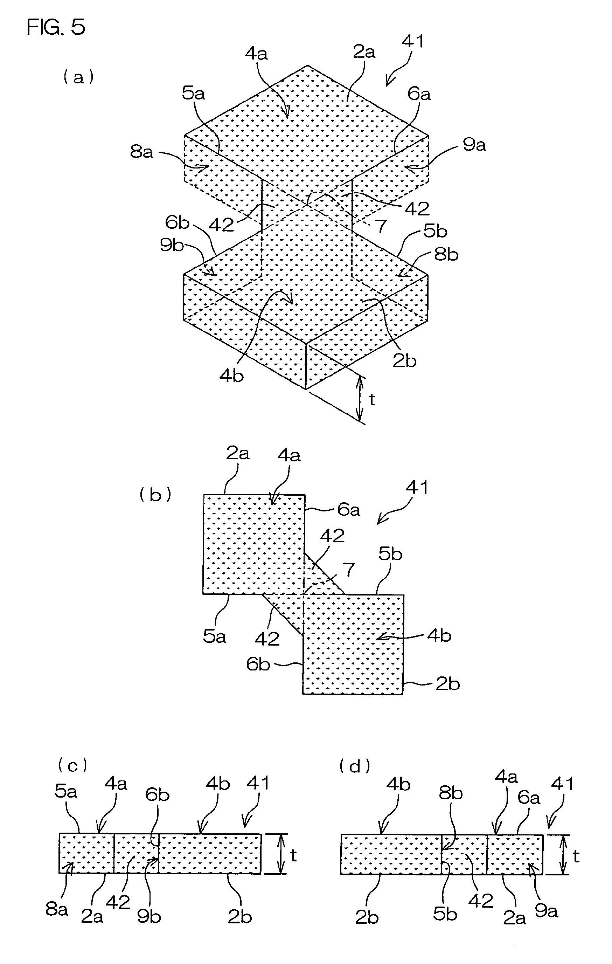

Figs. 5(a), 5(b), 5(c) and 5(d) are a perspective view, a plan view, a front view and a right side view, respectively, of a marker 41 according to further another embodiment of the present invention.

Features of the marker 41 will be described in comparison with the marker 1 shown in Figs. 1(a) to 1(d). The first plate member 2a and the second plate member 2b are disposed with their corners 7 opposed to each other, and connection portions 42 connect these two plate members 2a, 2b. Here, the plate member 2a, the plate member 2b and the connection portions 42 are unitarily formed of an imageable material. The connection potions 42 also each have a thickness t, and upper surfaces of the connection portions 42 are horizontally flush with the major surfaces 4a, 4b.

The marker 41 includes no complementary member, but has empty regions in which neither the plate members 2a, 2b nor the connection portions 42 are present.

Where the marker 41 is used for imaging a human body by means of a medical imaging apparatus, the major surfaces 4a, 4b, the straight line 5 (edges 5a+5b), the straight line 6 (edges 6a+6b), the side surfaces 8a, 8b and the side surfaces 9a, 9b serve as image positioning references. This will be described later in detail.

The marker 41 has substantially the same construction as the marker 1 described with reference to Figs. 1(a) to 1(d), except that the marker 41 includes the connection portions 42 and includes no complementary member. Therefore, like components are denoted by like reference characters, and duplicate description is omitted.

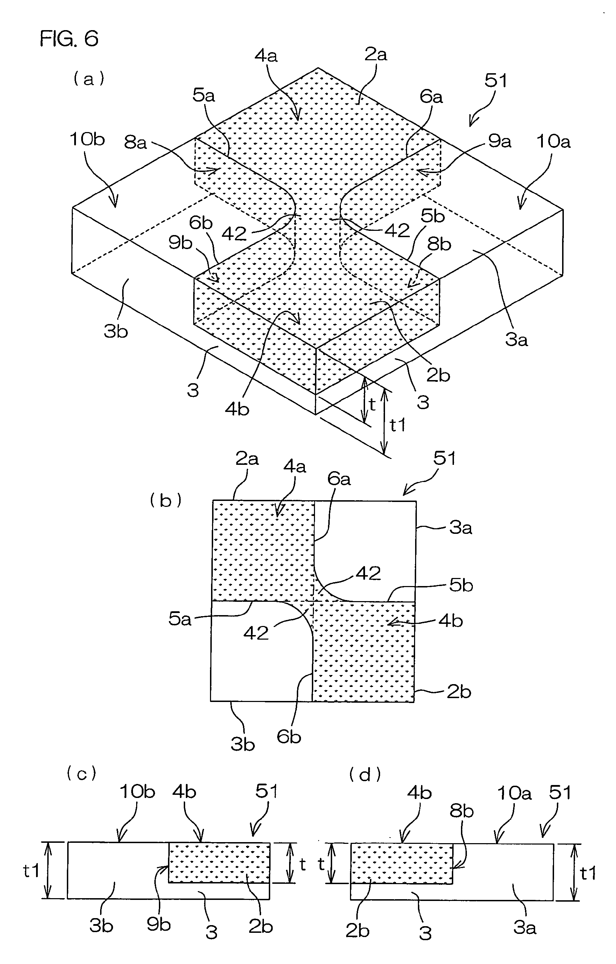

Figs. 6(a), 6(b), 6(c) and 6(d) are a perspective view, a plan view, a front view and a right side view, respectively, of a marker 51 according to still another embodiment of the present invention.

The marker 51 is different from the marker 41 shown in Figs. 5(a) to 5(d) in that outer edges of the connection portions 42 are not straight but curved, and a complementary member 3 fills the empty regions and covers the rear surfaces of the plate members 2a, 2b.

Even if the connection portions 42 each have an arcuate or curved contour as shown in Fig. 6(b), there is no problem in positioning the marker 51. This is because the plate members 2a, 2b connected by the connection portions 42 respectively have edges 5a, 5b defining parts of the straight line 5, and edges 6a, 6b defining parts of the straight line 6. Further, the side surfaces 8a, 8b, 9a, 9b are disposed perpendicularly to the major surfaces 4a, 4b with their boundary edges defined by the edges 5a, 5b, 6a, 6b.

The marker 51 has a rectangular-parallelepiped form as a whole, because the complementary members 3, 3a, 3b composed of the non-imageable material fill a region in which the two major surfaces 4a, 4b are absent, and cover the rear surfaces of the plate members 2a, 2b. Therefore, the marker 51 is easy to handle.

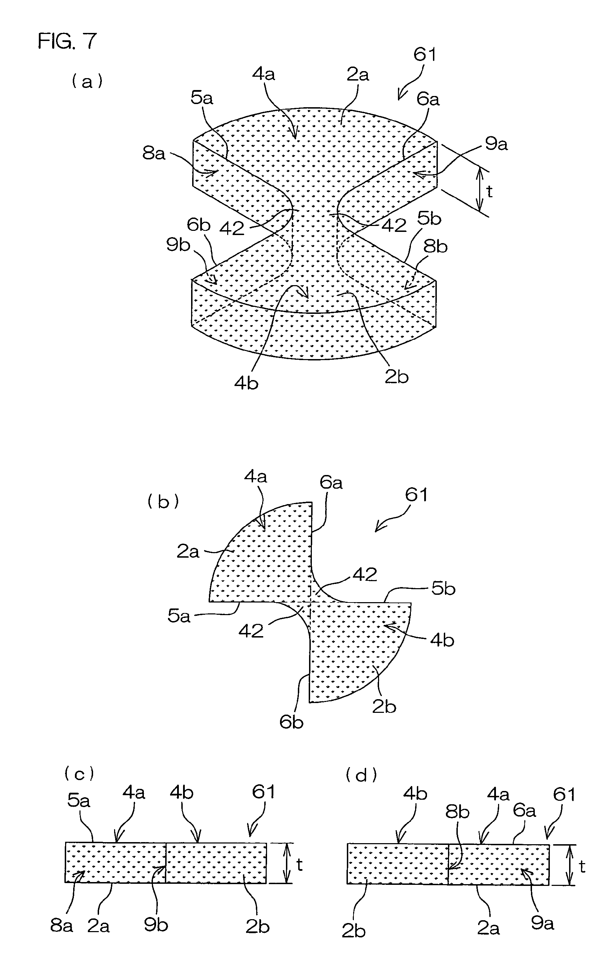

Figs. 7(a), 7(b), 7(c) and 7(d) are a perspective view, a plan view, a front view and a right side view, respectively, of a marker 61 according to further another embodiment of the present invention.

Features of the marker 61 shown in Figs. 7 (a) to 7(d) are that two plate members 2a, 2b each having a thickness t are connected to each other by connection portions 42 as in the marker 41 shown in Figs. 5(a) to 5(d) and respectively have major surfaces 4a, 4b horizontally flush with each other, that the contour of the marker 61 is not linear but arcuate, and that the empty regions are not filled with the complementary members of the non-imageable material.

Like the aforementioned markers, the marker 61 has the major surfaces 4a, 4b, edges 5a, 5b, 6a, 6b defining parts of straight lines, and side surfaces 8a, 8b, 9a, 9b perpendicular to the major surfaces 4a, 4b.

The marker 61 can have a desired outer shape.

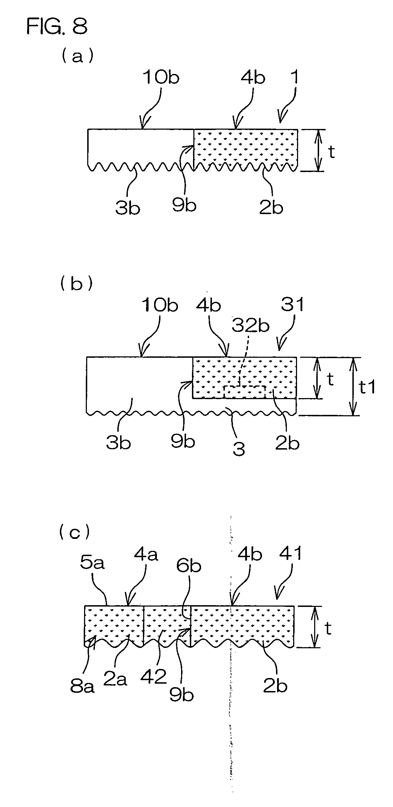

Figs. 8(a) to 8(c) are diagrams for explaining the fact that the rear surfaces of the markers 1, 31, 41 previously described may each have a desired non-planar geometry. Fig. 8(a) is a modification of Fig. 1(c), and Fig. 8(b) is a modification of Fig. 4(c). Further, Fig. 8(c) is a modification of Fig. 5(c).

As shown in Figs. 8(a) to 8(c), the rear surfaces of the markers may be non-planar, and each have a desired geometry. In this case, undulations formed in a desired geometry on the rear surfaces of the markers are preferably rounded.

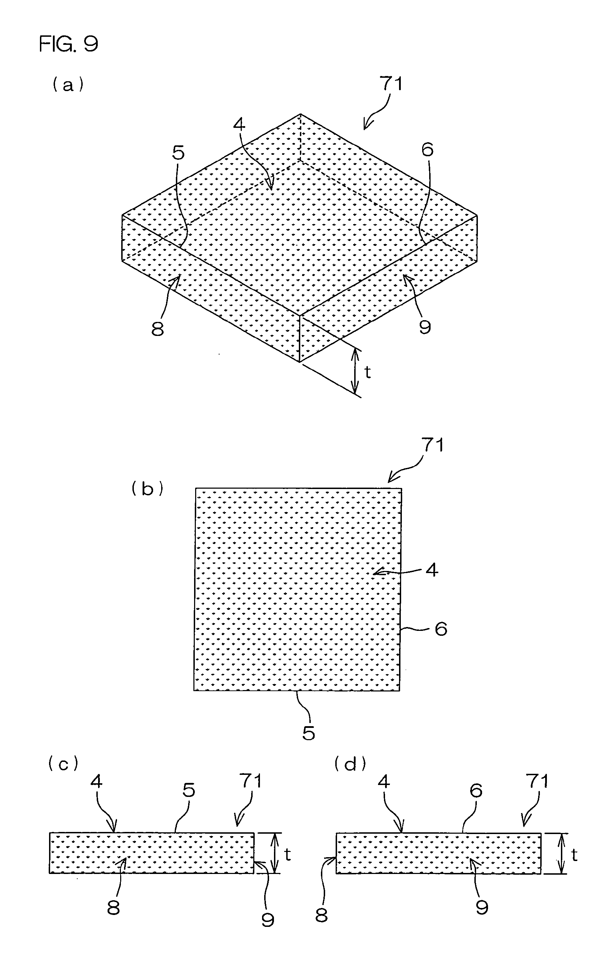

Figs. 9(a), 9(b), 9(c) and 9(d) are a perspective view, a plan view, a front view and a right side view, respectively, of a marker 71 according to still another embodiment of the present invention. The marker 71 shown in Figs. 9(a) to 9(d) are composed of an imageable material, and has a thin rectangular-parallelepiped form having a thickness t. The marker 71 has a major surface 4 defined on its upper surface, a side surface 8 perpendicular to the major surface 4 with its boundary edge defined by an edge 5, and a side surface 9 perpendicular to the major surface 4 with its boundary edge defined by an edge 6.

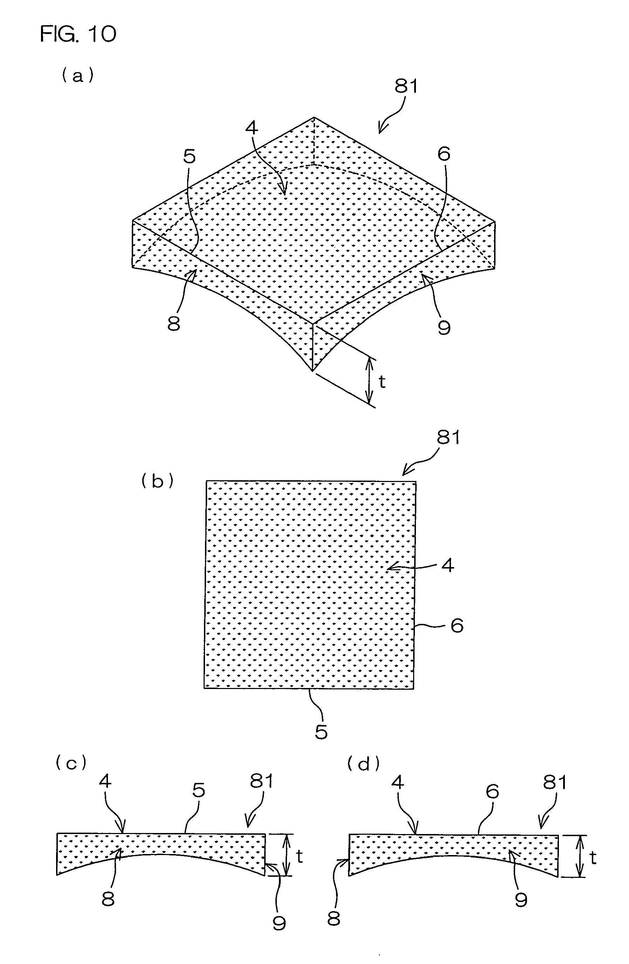

Figs. 10(a), 10(b), 10(c) and 10(d) are a perspective view, a plan view, a front view and a right side view, respectively, of a marker 81 according to further another embodiment of the present invention.

The marker 81 shown in Figs. 10(a) to 10(d) are different from the marker 71 shown in Figs. 9(a) to 9(d) in that its bottom surface are concavely curved as seen from the front side. The concavely curved geometry of the bottom surface of the marker 81 is advantageous for easy fitting of the marker on a predetermined site of a human body.

The marker 81 also has a major surface 4, edges 5, 6 defined by orthogonal straight lines, a side surface 8 perpendicular to the major surface 4 with its boundary edge defined by the edge 5, and a side surface 9 perpendicular to the major surface 4 with its boundary edge defined by the edge 6, which are all essential as positioning references.

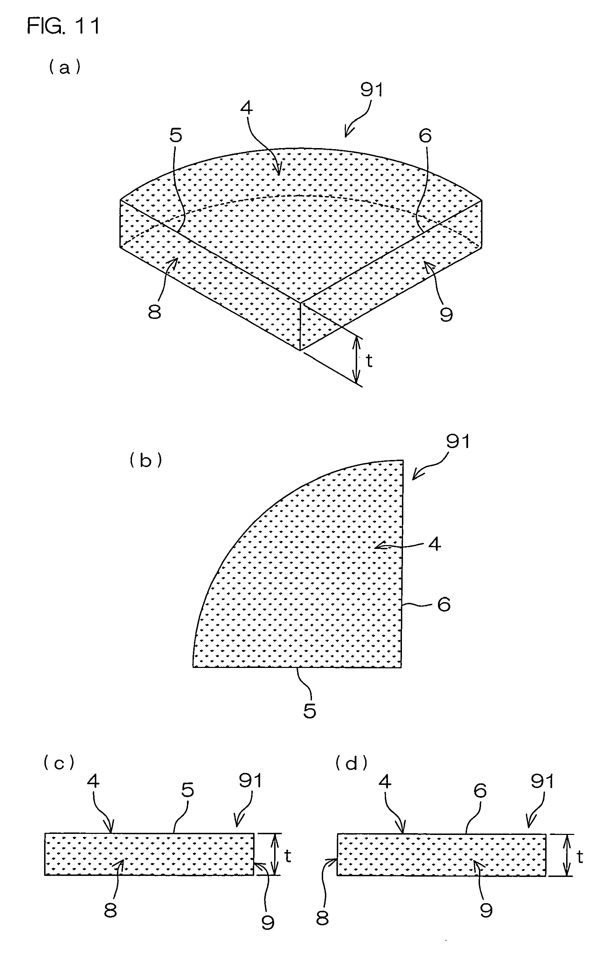

Figs. 11(a), 11(b), 11(c) and 11(d) are a perspective view, a plan view, a front view and a right side view, respectively, of a marker 91 according to still another embodiment of the present invention.

The marker 91 shown in Figs. 11(a) to 11(d) are a plate member entirely composed of an imageable material and having a thickness t, and has a flat major surface 4, edges 5, 6 defined by orthogonal straight lines and defining parts of the contour of the major surface 4, and side surfaces 8, 9 perpendicular to the major surface 4 with their boundary edges defined by the edges 5, 6. The marker 91 is merely required to have these positioning elements and, therefore, the other portion may have a curved geometry as shown in Figs. 11(a) and 11(b).

Next, description will be given to how to achieve image positioning based on a marker image when an actual entity fitted with an inventive marker is imaged.

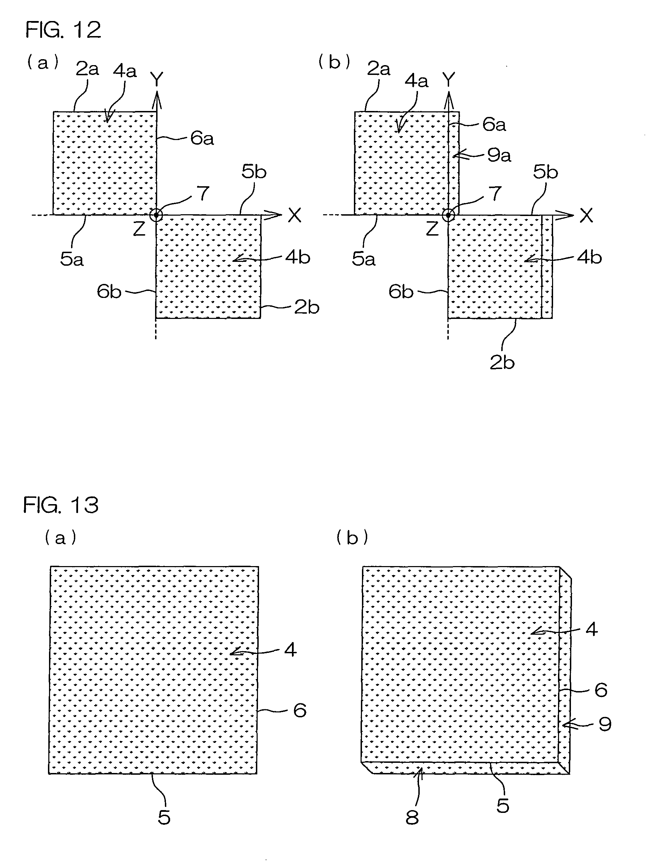

It is herein assumed that a human body fitted with the marker 1 shown in Figs. 1(a) to 1(d) are imaged. The marker 1 is displayed as a shadow image on an image captured through the imaging. The orientation of the image is adjusted so that the shadow image of the marker 1 is displayed as shown in Fig. 12(a). In this state, the image is such that the marker 1 is seen in plan, i.e., the edges 5a, 5b are displayed as aligned on a straight line, and the edges 6a, 6b are displayed as aligned on another straight line. Therefore, it is possible to define a Z-axis by an axis extending through a vertex 7 perpendicularly to a paper face, to define an X-axis by the straight line 5a, 5b, and to define a Y-axis by the straight line 6a, 6b.

If the image is oriented in an incorrect direction, the side surface 8a and/or 9a or the side surface 8b and/or 9b of the marker 1 is visible as shown in Fig. 12(b), making it possible to detect that the marker is not viewed from right above with the Z-axis being tilted.

Where the marker 71 described with reference to Figs. 9(a) to 9(d) are used, the coordinate system can also be defined in the same manner. That is, when a shadow image of the marker 71 is displayed as having a rectangular shape as shown in Fig. 13(a) in an image captured through the imaging with the marker 71 attached, the Z-axis is defined by an axis extending perpendicularly to the straight lines defined by the edges 5, 6.

If the image is not oriented along the Z-axis, shadows of the side surface 8 and/or the side surface 9 appear along the edges 5, 6 as shown in Fig. 13(b).

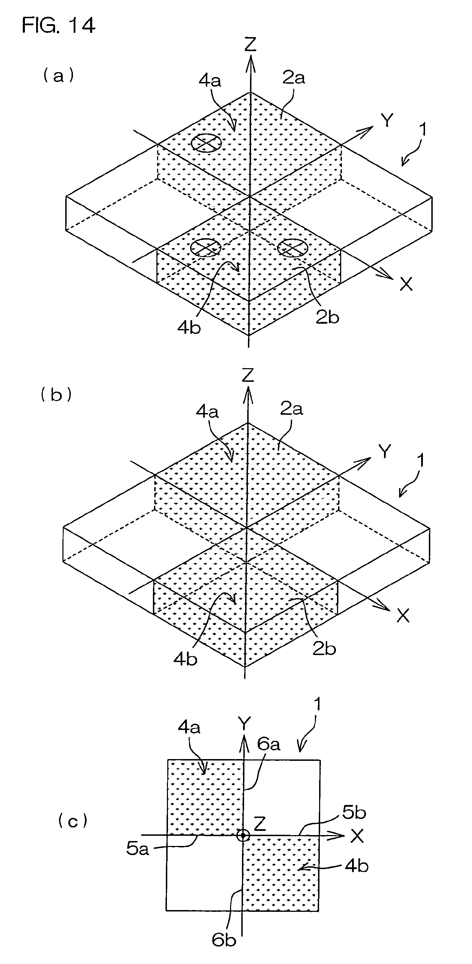

Figs. 14(a) to 14(c) are diagrams for explaining another method of defining the coordinate system based on a marker image.

As shown in Fig. 14(a), three points are specified on the major surfaces 4a, 4b of the plate members 2a, 2b displayed on an image captured through the imaging, and an axis extending perpendicularly to a plane containing the three points is defined as a positional coordinate axis, i.e., a Z-axis (Fig. 14(b)). Then, the captured image is rotated so that the marker image is viewed along the defined Z-axis. Thus, a straight line 5a, 5b and a straight line 6a, 6b in the marker image are defined as an X-axis and a Y-axis, respectively, whereby a three-dimensional coordinate system is defined (Fig. 14(c)).

The position of the marker fitted on an actual entity (a human body or a human body model) can be detected based on the major surfaces 4a, 4b, the edges 5a, 5b, 6a, 6b and the side surfaces 8a, 8b, 9a, 9b of the marker, whereby the actual entity can be correlated with the captured image.

Even with the marker 71 described with reference to Figs. 9(a) to 9(d), the Z-axis can be defined by specifying the three points on the major surface, and the X-axis and the Y-axis can be defined based on the edges 5, 6.

Next, how to correlate the captured image with the actual entity with the use of the inventive marker for the implantation of a dental implant will be described by way of a specific example.

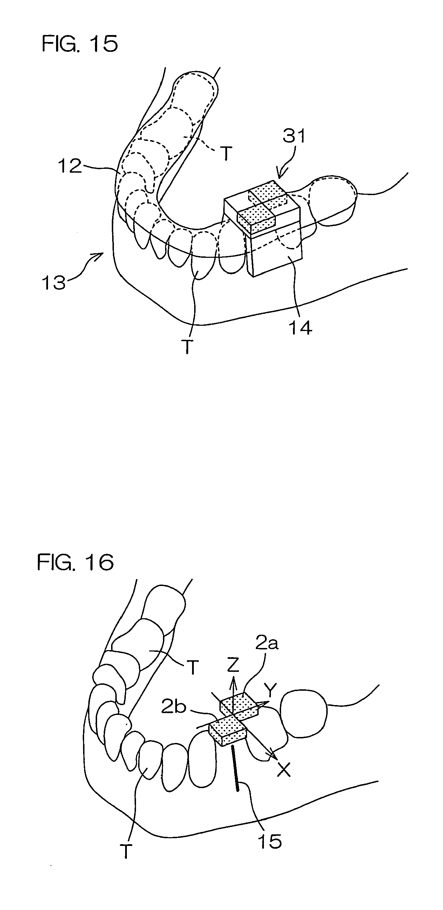

Fig. 15 is a perspective view illustrating a stent 12 fitted on an actual entity (dental arch model) and marked with the marker 31 according to the embodiment of the present invention. The stent 12 is entirely composed of a non-imageable material, and fitted on a plurality of teeth T of the dental arch model 13. The marker 31 is fixed to a predetermined position of the stent 12. More specifically, the complementary members of the marker 31 are partly fixed to the non-imageable material of the stent 12.

In Fig. 15, side plates 14 are provided in association with the marker 31 as extending downward, but the side plates 14 may be obviated.

Where the dental arch model 13 on which the stent 12 marked with the marker 31 shown in Fig. 15 is attached is imaged by means of a medical imaging apparatus such as CT, an image shown in Fig. 16 is provided. The image is constructed as a three-dimensional image, which can be seen in any desired direction. In the image, the stent 12 is not displayed, but only teeth T, a lower jaw bone and the plate members 2a, 2b of the marker 31 are displayed.

In image diagnosis, an implant implantation position and direction are determined based on the image. In Fig. 16, a bold line 15 indicates the implant implantation position and direction determined through the diagnosis.

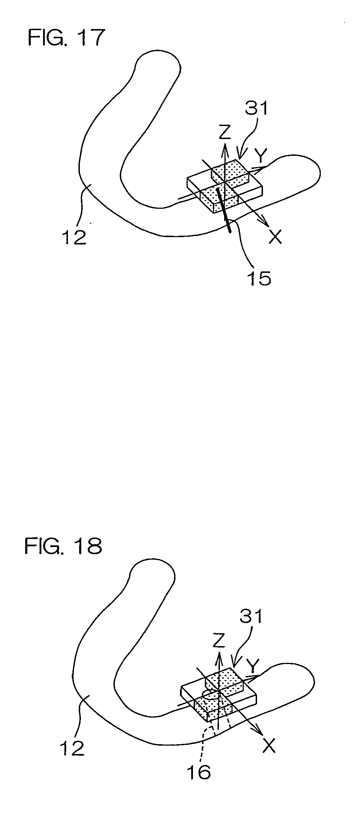

Then, the XYZ coordinate system for the marker is determined based on the shadow images of the plate members 2a, 2b of the marker 31 in the image shown in Fig. 16, and the implant implantation position and direction determined through the diagnosis are mapped onto the stent 12 based on the XYZ coordinate system thus determined (Fig. 17).

In turn, as shown in Fig. 18, the implant implantation position and direction 15 mapped onto the stent are determined by a three-dimensional measurement apparatus or the like, and a mark (a guide channel 16 or the like) is formed in the stent. Thus, a guide as shown in Fig. 18 is produced.

Subsequently, the guide is attached to the actual entity, and an implant implantation treatment is performed.

It should be understood that the present invention be not limited to the markers according to the embodiments described above, but various modifications may be made within the scope of the claims. For example, an additional member formed of a non-imageable material and having a desired configuration may be provided on any of the markers.

## Claims

1. A medical imaging marker comprising a plate member of an imageable material which includes:
two flat major surfaces located symmetrically about an intersection of two orthogonal straight lines and each having edges defined by the straight lines; and
two pairs of side surfaces disposed perpendicularly to the respective major surfaces with their boundary edges defined by at least parts of the straight lines.

2. A medical imaging marker as set forth in claim 1, further comprising a complementary member of a non-imageable material which fills a region in which the two major surfaces are absent, and has a flat surface continuous to the two major surfaces.

3. A medical imaging marker as set forth in claim 2, wherein the complementary member entirely covers a back surface of the plate member of the imageable material opposite from the two major surfaces.

4. A medical imaging marker as set forth in claim 1 or 2, which has a rectangular contour as a whole as seen from above the major surfaces perpendicularly to the major surfaces.

5. A medical imaging marker as set forth in claim 2 or 3, which has a round contour as a whole as seen from above the major surfaces perpendicularly to the major surfaces.

6. A medical imaging marker as set forth in any of claims 1 to 5, wherein the plate member includes a connection portion which connects the two major surfaces in a region including the intersection of the two straight lines.

7. A medical imaging marker as set forth in any of claims 1 to 6, wherein the back surface opposite from the major surfaces has a given non-planar geometry.

8. A planar medical imaging marker entirely composed of an imageable material, and comprising:
a flat major surface having at least two orthogonal edges; and
a pair of side surfaces extending perpendicularly from the two orthogonal edges of the major surface with their boundary edges defined by the two orthogonal edges.
